# EUROPEAN PATENT APPLICATION

(11) **EP 1 661 918 A1**
(43) Date of publication of application: **31.05.2006**
(21) Application number: 04772867.0
(22) Date of filing: 31.08.2004
(51) Int. Cl.: C08F 8/00, C08G 8/36, C07B 51/00, G03F 7/039, C07C 41/52, C07C 43/303, C07C 67/14, C07C 69/14

(54) **METHOD FOR PRODUCING COMPOUND HAVING ACID-LABILE GROUP**

(30) Priority: 03.09.2003 JP 2003311084
(71) Applicant: Kyowa Hakko Chemical Co., Ltd., Tokyo 103-0022 (JP)
(72) Inventor: MATSUOKA, Hiroshi, Yokkaichi Res. Lab., Yokkaichi-shi, Mie 510-8502 (JP); YAMANO, Junzo Yokkaichi Res. Lab., Yokkaichi-shi, Mie 510-8502 (JP); ITO, Katsuhiro Yokkaichi Res. Lab., Yokkaichi-shi, Mie 510-8502 (JP); NUMAZAKI, Ryo Yokkaichi Res. Lab., Yokkaichi-shi, Mie 510-8502 (JP); SHIMIZU, Ikuo Yokkaichi Res. Lab., Yokkaichi-shi, Mie 510-8502 (JP)
(74) Representative: Casalonga, Axel
(86) International application number: PCT/JP2004/012918
(87) International publication number: WO 2005/023880

(57) **Abstract**

The present invention provides a process for producing a compound having a group represented by general formula (II): 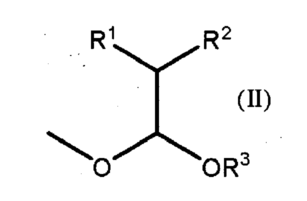 (wherein R¹, R², and R³ may be the same or different, and each represent a substituted or unsubstituted alkyl, a substituted or unsubstituted aryl, or a substituted or unsubstituted aralkyl, or R¹ and R² may bind to each other to form an alicyclic hydrocarbon ring together with the adjacent carbon atoms, or R² and R³ may bind to each other to form a alicyclic heterocyclic ring together with the adjacent O-C-C that may have a substituent), which comprises allowing a compound having a hydroxyl group to react with halogenated alkyl ether represented by general formula (I): 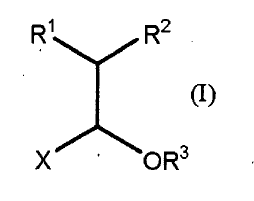 (wherein R¹, R², and R³ are the same as those defined above, respectively and X represents a halogen atom).

## Description

### Technical Field

The present invention relates to a process for producing a compound having an acid-labile group, which is useful for chemical amplification resist compositions, paints, or the like.

### Background Art

Polymers having an acetal or a hemiacetal ester derived from an alkyl vinyl ether are useful for chemical amplification resist compositions, paints, or the like because elimination of a group derived from the alkyl vinyl ether by heat, an acid catalyst, or the like easily occurs.

As processes for producing a polymer having an acetal or a hemiacetal ester derived from an alkyl vinyl ether, a process for allowing, for example, an alkenyl ether compound to react with a polymer having a hydroxyl group in the presence of a suitable acid (such as sulfuric acid, hydrochloric acid, or p-toluenesulfonic acid), if necessary (for example, Japanese Published Unexamined Patent Application No. 249682/1993 and PCT Publication No. WO03/006407) is known. However, such a process has a problem of long reaction time.

A process for allowing, for example, a halogenated alkyl ether to react with a polymer having a hydroxyl group is also known (for example, Japanese Published Unexamined Patent Application No. 222507/1999). However, when 1-chloroethyl ethyl ether is allowed to react with a polymer having a hydroxyl group or the like, there have been problems of, for example, coloring of the reaction solution and the production of a byproduct such as a polymer of 1-chloroethyl ethyl ether. In addition, when a polymer having a hydroxyl group, which is subjected to acetalization using 1-chloroethyl ethyl ether, is used in a chemical amplification resist composition, thermal stability of the acetal is unsatisfactory in a step of pre-baking, which is conducted for the purpose of removing an organic solvent when the composition is applied on a silicon wafer by spin coating, and stability of the acetal during a long-term storage is also unsatisfactory.

### Disclosure of Invention

The present invention provides the following [1] to [17].
[1] A process for producing a compound having a group represented by general formula (II): 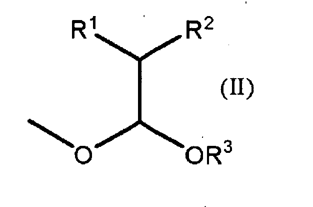 (wherein R¹, R², and R³ may be the same or different, and each represent a substituted or unsubstituted alkyl, a substituted or unsubstituted aryl, or à substituted or unsubstituted aralkyl, or R¹ and R² may bind to each other to form an alicyclic hydrocarbon ring together with the adjacent carbon atoms, or R² and R³ may bind to each other to form a alicyclic heterocyclic ring together with the adjacent O-C-C that may have a substituent), which comprises allowing a compound having a hydroxyl group to react with a halogenated alkyl ether represented by general formula (I): 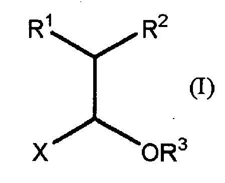 (wherein R¹, R², and R³ are the same as those defined above, respectively and X represents a halogen atom).
[2] A process for producing a compound having a group represented by general formula (IIa): 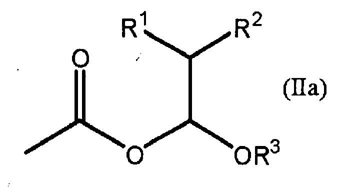 (wherein R¹, R², and R³ are the same as those defined above, respectively), which comprises allowing a compound having a carboxyl group to react with a halogenated alkyl ether represented by general formula (I): 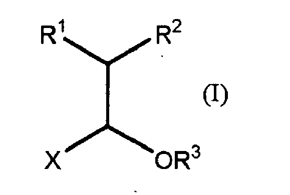 (wherein R¹, R², R³, and X are the same as those defined above, respectively).
[3] A polyhydroxystyrene derivative wherein a hydroxyl group of the polyhydroxystyrene is substituted with a group represented by general formula (II): 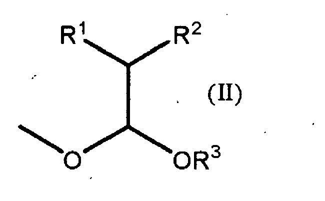 (wherein R¹, R², and R³ are the same as those defined above, respectively).
[4] A phenol novolak resin derivative wherein a hydroxyl group of the phenol novolak resin is substituted with a group represented by general formula (II): 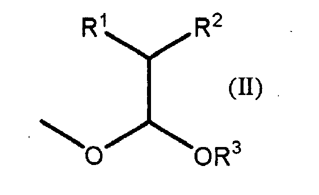 (wherein R¹, R², and R³ are the same as those defined above, respectively).
[5] A protective agent for a hydroxyl group, which comprises a halogenated alkyl ether represented by general formula (I): 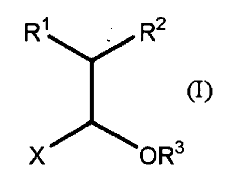 (wherein R¹, R², R³, and X are the same as those defined above, respectively).
[6] A protective agent for a carboxyl group, which comprises a halogenated alkyl ether represented by general formula (I): 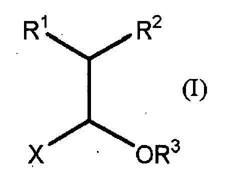 (wherein R¹, R², and R³ are the same as those defined above, respectively).
[7] A chemical amplification resist composition which comprises a polyhydroxystyrene derivative in which a hydroxyl group of a polyhydroxystyrene is substituted with a group represented by general formula (II): 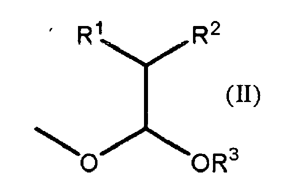 (wherein R¹, R², and R³ are the same as those defined above, respectively) and a photoacid generator.
[8] A chemical amplification resist composition which comprises a phenol novolak resin derivative in which a hydroxyl group of a phenol novolak resin is substituted with a group represented by general formula (II): 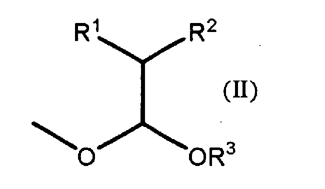 (wherein R¹, R², and R³ are the same as those defined above, respectively) and a photoacid generator.
[9] A process for producing a compound having a group represented by general formula (V): 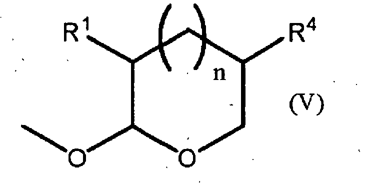 (wherein R¹ is the same as that defined above, R⁴ represents a hydrogen atom, a substituted or unsubstituted alkyl, a substituted or unsubstituted aryl, or a substituted or unsubstituted aralkyl, and n represents 0 or 1), which comprises allowing a compound having a hydroxyl group to react with a tetrahydrofuran or tetrahydropyran compound represented by general formula (IV): 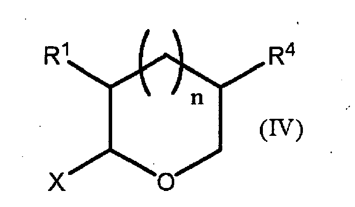 (wherein R¹, R⁴, n, and X are the same as those defined above, respectively).
[10] A process for producing a compound having a group represented by general formula (Va): 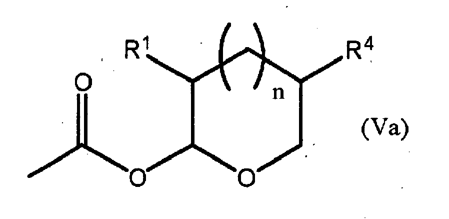 (wherein R¹, R⁴, and n are the same as those defined above,' respectively), which comprises allowing a compound having a carboxyl group to react with a tetrahydrofuran or tetrahydropyran compound represented by general formula (IV): 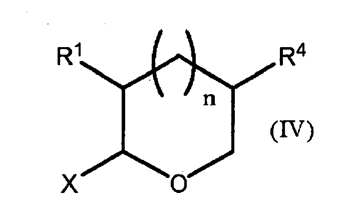 (wherein R¹, R⁴, and n are the same as those defined above, respectively).
[11] A polyhydroxystyrene derivative wherein a hydroxyl group of the polyhydroxystyrene is substituted with a group represented by general formula (V): 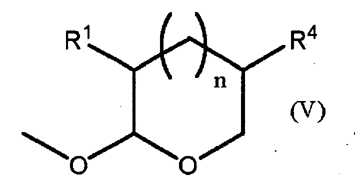 (wherein R¹, R⁴, and n are the same as those defined above, respectively).
[12] A phenol novolak resin derivative wherein a hydroxyl group of the phenol novolak resin is substituted with a group represented by general formula (V): 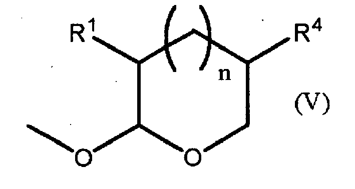 (wherein R¹, R⁴, and n are the same as those defined above, respectively).
[13] A protective agent for a hydroxyl group, which comprises a tetrahydrofuran or tetrahydropyran compound represented by general formula (IV): 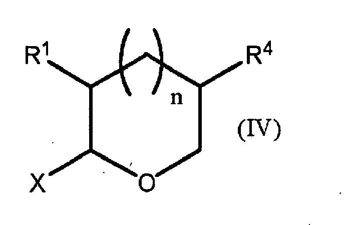 (wherein R¹, R⁴, and n are the same as those defined above, respectively).
[14] A protective agent for a carboxyl group, which comprises a tetrahydrofuran or tetrahydropyran compound represented by general formula (IV): 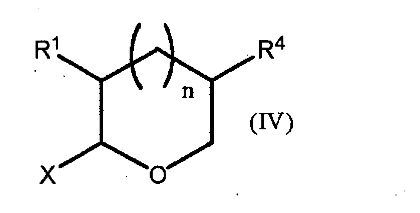 (wherein R¹, R⁴, and n are the same as those defined above, respectively).
[15] A chemical amplification resist composition which comprises a polyhydroxystyrene derivative in which a hydroxyl group of a polyhydroxystyrene is substituted with a group represented by general formula (V): 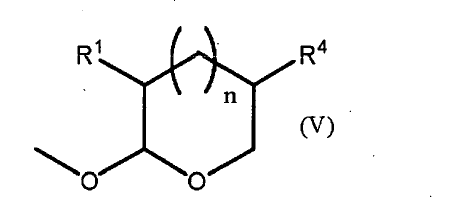 (wherein R¹, R⁴, and n are the same as those defined above, respectively) and a photoacid generator.
[16] A chemical amplification resist composition which comprises a phenol novolak resin derivative in which a hydroxyl group of a phenol novolak resin is substituted with a group represented by general formula (V): 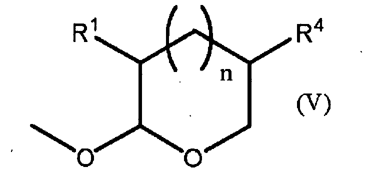 (wherein R¹, R⁴, and n are the same as those defined above, respectively) and a photoacid generator.
[17] A tetrahydrofuran or tetrahydropyran compound represented by general formula (IV): 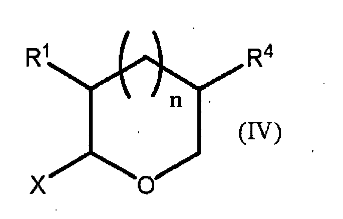 (wherein R¹, R⁴, and n are the same as those defined above, respectively).

Hereinafter, the halogenated alkyl ether represented by general formula (I) may also be referred to as compound (I), the compound having a group represented by general formula (II) may also be referred to as compound (II), the compound having a group represented by general formula (IIa) may also be referred to as compound (IIa), the tetrahydrofuran or tetrahydropyran compound represented by general formula (IV) may also be referred to as compound (IV), the compound having a group represented by general formula (V) may also be referred to as compound (V), and the compound having a group represented by general formula (Va) may also be referred to as compound (Va).

In the definition of each group in the general formulae, examples of the alkyl include linear or branched alkyls each having 1 to 18 carbons, and specific examples thereof include methyl, ethyl, propyl, isopropyl, butyl, isobutyl, sec-butyl, tert-butyl, pentyl, hexyl, heptyl, octyl, nonyl, decyl, dodecyl, octadecyl and the like. Among these, an alkyl having 1 to 6 carbons is preferred and an alkyl having 1 to 3 carbons is more preferred.

Examples of the aryl include phenyl, naphthyl and the like.

Examples of the aralkyl include aralkyls having 7 to 15 carbons and specific examples thereof include benzyl, phenethyl, naphthylmethyl, naphthylethyl and the like.

Examples of the alicyclic hydrocarbon ring formed by binding R¹ and R² together with the adjacent carbon atoms include alicyclic hydrocarbon rings each having 3 to 8 carbons that may be saturated or unsaturated. Specific examples thereof include cyclopropane ring, cyclobutane ring, cyclopentane ring, cyclohexane ring, cycloheptane ring, cyclooctane ring, cyclopentene ring, 1,3-cyclopentadiene ring, cyclohexene ring, cyclohexadiene ring and the like.

Examples of the alicyclic heterocyclic ring formed by binding R² and R³ together with the adjacent O-C-C include alicyclic heterocyclic rings each having 5 to 8 carbons and at least one oxygen atom. Specific examples thereof include oxolane ring, oxane ring, oxepane ring, and oxocane ring.

Examples of the halogen atom include atoms of fluorine, chlorine, bromine, and iodine. Among these, chlorine atom is preferred.

Examples of the substituent in the substituted alkyl include alkoxy, alkanoyl, cyano, nitro, halogen atom, alkoxycarbonyl and the like.

Examples of the substituent in the substituted aryl and the substituted aralkyl include alkyl, alkoxy, alkanoyl, cyano, nitro, a halogen atom, alkoxycarbonyl and the like.

Examples of the substituent in the optionally substituted alicyclic heterocyclic ring include substituted or unsubstituted alkyl (examples of the substituent in the substituted alkyl include alkoxy, alkanoyl, cyano, nitro, a halogen atom, alkoxycarbonyl and the like), substituted or unsubstituted aryl, and substituted or unsubstituted aralkyl (examples of the substituent in the substituted aryls and the substituted aralkyl include alkyl, alkoxy, alkanoyl, cyano, nitro, a halogen atom, alkoxycarbonyl and the like).

In the definition of the substituent, examples of the alkyl moiety of alkyl, alkoxy, and alkoxycarbonyl include the same alkyl as those described as examples in the above alkyl. Examples of the alkanoyl include linear or branched alkanoyl each having 1 to 7 carbons, and specific examples thereof include formyl, acetyl, propionyl, butyryl, isobutyryl, valeryl, isovaleryl, pivaloyl, hexanoyl, heptanoyl and the like. Examples of the halogen atom include the same halogen atoms as those described above.

Specific examples of compound (I) include 1-chloro-1-methoxy-2-methylpropane, 1-chloro-1-ethoxy-2-methylpropane, 1-chloro-1-propoxy-2-methylpropane, 1-chloro-1-isopropoxy-2-methylpropane, 1-butoxy-1-chloro-2-methylpropane, 1-chloro-1-isobutoxy-2-methylpropane, 1-(tert-butoxy)-1-chloro-2-methylpropane, 1-chloro-1-pentyloxy-2-methylpropane, 1-chloro-1-isopentyloxy-2-methylpropane, 1-chloro-1-neopentyloxy-2-methylpropane, 1-chloro-1-(tert-pentyloxy)-2-methylpropane, 1-chloro-1-hexyloxy-2-methylpropane, 1-chloro-1-isohexyloxy-2-methylpropane, 1-chloro-1-(2-ethylhexyloxy)-2-methylpropane, 1-chloro-1-heptyloxy-2-methylpropane, 1-chloro-1-octyloxy-2-methylpropane, 1-chloro-1-nonyloxy-2-methylpropane, 1-chloro-1-decanyloxy-2-methylpropane, 1-chloro-1-dodecanyloxy-2-methylpropane, 1-chloro-1-octadecanyloxy-2-methylpropane, 1-chloro-1-methoxy-2-methylbutane, 1-chloro-1-ethoxy-2-methylbutane, 1-chloro-1-propoxy-2-methylbutane, 1-chloro-1-isopropoxy-2-methylbutane, 1-butoxy-1-chloro-2-methylbutane, 1-chloro-1-isobutoxy-2-methylbutane, 1-(tert-butoxy)-1-chloro-2-methylbutane, 1-chloro-1-pentyloxy-2-methylbutane, 1-chloro-1-isopentyloxy-2-methylbutane, 1-chloro-1-neopentyloxy-2-methylbutane, 1-chloro-1-(tert-pentyloxy)-2-methylbutane, 1-chloro-1-hexyloxy-2-methylbutane, 1-chloro-1-isohexyloxy-2-methylbutane, 1-chloro-1-(2-ethylhexyloxy)-2-methylbutane, 1-chloro-1-heptyloxy-2-methylbutane, 1-chloro-1-octyloxy-2-methylbutane, 1-chloro-1-nonyloxy-2-methyl-butane, 1-chloro-1-decanyloxy-2-methylbutane, 1-chloro-1-dodecanyloxy-2-methylbutane, 1-chloro-1-octadecanyloxy-2-methylbutane, 1-chloro-1-methoxy-2-ethylbutane, 1-chloro-1-ethoxy-2-ethylbutane, 1-chloro-1-propoxy-2-ethylbutane, 1-chloro-1-isopropoxy-2-ethylbutane, 1-butoxy-1-chloro-2-ethylbutane, 1-chloro-1-isobutoxy-2-ethylbutane, 1-(tert-butoxy)-1-chloro-2-ethylbutane, 1-chloro-1-pentyloxy-2-ethylbutane, 1-chloro-1-isopentyloxy-2-ethylbutane, 1-chloro-1-neopentyloxy-2-ethylbutane, 1-chloro-1-(tert-pentyloxy)-2-ethylbutane, 1-chloro-1-hexyloxy-2-ethylbutane, 1-chloro-1-isohexyloxy-2-ethylbutane, 1-chloro-1-(2-ethylhexyloxy)-2-ethylbutane, 1-chloro-1-heptyloxy-2-ethylbutane, 1-chloro-1-octyloxy-2-ethylbutane, 1-chloro-1-nonyloxy-2-ethylbutane, 1-chloro-1-decanyloxy-2-ethylbutane, 1-chloro-1-dodecanyloxy-2-ethylbutane, 1-chloro-1-octadecanyloxy-2-ethylbutane, 1-chloro-1-(2-methoxyethoxy)-2-methylpropane, 1-chloro-1-(2-ethoxyethoxy)-2-methylpropane, 1-(2-butoxyethoxy)-1-chloro-2-methylpropane, 1-chloro-1-(2= methoxyethoxy)-2-methylbutane, 1-chloro-1-(2-ethoxyethoxy)-2-methylbutane, 1-(2-butoxyethoxy)-1-chloro-2-methylbutane, 1-chloro-1-(2-methoxyethoxy)-2-ethylbutane, 1-chloro-1-(2-ethoxyethoxy)-2-ethylbutane, 1-(2-butoxyethoxy)-1-chloro-2-ethylbutane and the like. Among these, 1-chloro-1-methoxy-2-methylpropane is preferred. Compound (I) may be used as a single compound or a mixture of two or more compounds.

Specific examples of compound (IV) include 2-chloro-3-methyltetrahydrofuran, 2-chloro-3-ethyltetrahydrofuran, 2-chloro-3-propyltetrahydrofuran, 2-chloro-3-isopropyltetrahydrofuran, 2-chloro-3-butyltetrahydrofuran, 2-chloro-3-isobutyltetrahydrofuran, 2-chloro-3-(tert-butyl)tetrahydrofuran, 2-chloro-3-pentyltetrahydrofuran, 2-chloro-3-isopentyltetrahydrofuran, 2-chloro-3-neopentyltetrahydrofuran, 2-chloro-3-(tert-pentyl)tetrahydrofuran, 2-chloro-3-hexyltetrahydrofuran, 2-chloro-3-isohexyltetrahydrofuran, 2-chloro-3-(2-ethylhexyl)tetrahydrofuran, 2-chloro-3-heptyltetrahydrofuran, 2-chloro-3-octyltetrahydrofuran, 2-chloro-3-nonyltetrahydrofuran, 2-chloro-3-decanyltetrahydrofuran, 2-chloro-3-dodecanyltetrahydrofuran, 2-chloro-3-octadecanyltetrahydrofuran, 2-chloro-3,4-dimethyltetrahydrofuran, 2-chloro-3-ethyl-4-methyltetrahydrofuran, 2-chloro-3-propyl-4-methyltetrahydrofuran, 2-chloro-3-isopropyl-4-methyltetrahydrofuran, 2-chloro-3-butyl-4-methyltetrahydrofuran, 2-chloro-3-isobutyl-4-methyltetrahydrofuran, 2-chloro-3-(tert-butyl)-4-methyltetrahydrofuran, 2-chloro-3-pentyl-4-methyltetrahydrofuran, 2-chloro-3-isopentyl-4-methyltetrahydrofuran, 2-chloro-3-neopentyl-4-methyltetrahydrofuran, 2-chloro-3-(tert-pentyl)-4-methyltetrahydrofuran, 2-chloro-3-hexyl-4-methyltetrahydrofuran, 2-chloro-3-isohexyl-4-methyltetrahydrofuran, 2-chloro-3-(2-ethylhexyl)-4-methyltetrahydrofuran, 2-chloro-3-heptyl-4-methyltetrahydrofuran, 2-chloro-3-octyl-4-methyltetrahydrofuran, 2-chloro-3-nonyl-4-methyltetrahydrofuran, 2-chloro-3-decanyl-4-methyltetrahydrofuran, 2-chloro-3-dodecanyl-4-methyltetrahydrofuran, 2-chloro-3-octadecanyl-4-methyltetrahydrofuran, 2-chloro-3-methyl-4-ethyltetrahydrofuran, 2-chloro-3,4-diethyltetrahydrofuran, 2-chloro-3-propyl-4-ethyltetrahydrofuran, 2-chloro-3-isopropyl-4-ethyltetrahydrofuran, 2-chloro-3-butyl-4-ethyltetrahydrofuran, 2-chloro-3-isobutyl-4-ethyltetrahydrofuran, 2-chloro-3-(tert-butyl)-4-ethyltetrahydrofuran, 2-chloro-3-pentyl-4-ethyltetrahydrofuran, 2-chloro-3-isopentyl-4-ethyltetrahydrofuran, 2-chloro-3-neopentyl-4-ethyltetrahydrofuran, 2-chloro-3-(tert-pentyl)-4-ethyltetrahydrofuran, 2-chloro-3-hexyl-4-ethyltetrahydrofuran, 2-chloro-3-isohexyl-4-ethyltetrahydrofuran, 2-chloro-3-(2-ethylhexyl)-4-ethyltetrahydrofuran, 2-chloro-3-heptyl-4-ethyltetrahydrofuran, 2-chloro-3-octyl-4-ethyltetrahydrofuran, 2-chloro-3-nonyl-4-ethyltetrahydrofuran, 2-chloro-3-decanyl-4-ethyltetrahydrofuran, 2-chloro-3-dodecanyl-4-ethyltetrahydrofuran, 2-chloro-3-octadecanyl-4-ethyltetrahydrofuran, 2-chloro-3-methyltetrahydropyran, 2-chloro-3-ethyltetrahydropyran, 2-chloro-3-propyltetrahydropyran, 2-chloro-3-isopropyltetrahydropyran, 2-chloro-3-butyltetrahydropyran, 2-chloro-3-isobutyltetrahydropyran, 2-chloro-3-(tert-butyl)tetrahydropyran, 2-chloro-3-pentyltetrahydropyran, 2-chloro-3-isopentyltetrahydropyran, 2-chloro-3-neopentyltetrahydropyran, 2-chloro-3-(tert-pentyl)tetrahydropyran, 2-chloro-3-hexyltetrahydropyran, 2-chloro-3-isohexyltetrahydropyran, 2-chloro-3-(2-ethylhexyl)tetrahydropyran, 2-chloro-3-heptyltetrahydropyran, 2-chloro-3-octyltetrahydropyran, 2-chloro-3-nonyltetrahydropyran, 2-chloro-3-decanyltetrahydropyran, 2-chloro-3-dodecanyltetrahydropyran, 2-chloro-3-octadecanyltetrahydropyran, 2-chloro-3,5-dimethyltetrahydropyran, 2-chloro-3-ethyl-5-methyltetrahydropyran, 2-chloro-3-propyl-5-methyltetrahydropyran, 2-chloro-3-isopropyl-5-methyltetrahydropyran, 2-chloro-3-butyl-5-methyltetrahydropyran, 2-chloro-3-isobutyl-5-methyltetrahydropyran, 2-chloro-3-(tert-butyl)-5-methyltetrahydropyran, 2-chloro-3-pentyl-5-methyltetrahydropyran, 2-chloro-3-isopentyl-5-methyltetrahydropyran, 2-chloro-3-neopentyl-5-methyltetrahydropyran, 2-chloro-3-(tert-pentyl)-5-methyltetrahydropyran, 2-chloro-3-hexyl-5-methyltetrahydropyran, 2-chloro-3-isohexyl-5-methyltetrahydropyran, 2-chloro-3-(2-ethylhexyl)-5-methyltetrahydropyran, 2-chloro-3-heptyl-5-methyltetrahydropyran, 2-chloro-3-octyl-5-methyltetrahydropyran, 2-chloro-3-nonyl-5-methyltetrahydropyran, 2-chloro-3-decanyl-5-methyltetrahydropyran, 2-chloro-3-dodecanyl-5-methyltetrahydropyran, 2-chloro-3-octadecanyl-5-methyltetrahydropyran, 2-chloro-3-methyl-5-ethyltetrahydropyran, 2-chloro-3,5-diethyltetrahydropyran, 2-chloro-3-propyl-5-ethyltetrahydropyran, 2-chloro-3-isopropyl-5-ethyltetrahydropyran, 2-chloro-3-butyl-5-ethyltetrahydropytan, 2-chloro-3-isobutyl-5-ethyltetrahydropyran, 2-chloro-3-(tert-butyl)-5-ethyltetrahydropyran, 2-chloro-3-pentyl-5-ethyltetrahydropyran, 2-chloro-3-isopentyl-5-ethyltetrahydropyran, 2-chloro-3-neopentyl-5-ethyltetrahydropyran, 2-chloro-3-(tert-pentyl)-5-ethyltetrahydropyran, 2-chloro-3-hexyl-5-ethyltetrahydropyran, 2-chloro-3-isohexyl-5-ethyltetrahydropyran, 2-chloro-3-(2-ethylhexyl)-5-ethyltetrahydropyran, 2-chloro-3-heptyl-5-ethyltetrahydropyran, 2-chloro-3-octyl-5-ethyltetrahydropyran, 2-chloro-3-nonyl-5-ethyltetrahydropyran, 2-chloro-3-decanyl-5-ethyltetrahydropyran, 2-chloro-3-dodecanyl-5-ethyltetrahydropyran, and 2-chloro-3-octadecanyl-5-ethyltetrahydropyran. , Among these, 2-chloro-3-methyltetrahydrofuran, 2-chloro-3-methyltetrahydropyran, and 2-chloro-3,5-diethyltetrahydropyran are preferred. Compound (IV) may be used as a single compound or a mixture of two or more compounds.

Examples of the compound having a hydroxyl group include alcohols, phenols, and compounds each having a carboxyl group.

Examples of alcohol include e.g., monoalcohols such as methanol, ethanol, propanol, isopropyl alcohol, n-butanol, isobutanol, sec-butyl alcohol, pentanol, hexanol, heptanol, octanol, nonanol, decanol and benzyl alcohol; and polyhydric alcohols such as ethylene glycol, 1,3-propylene glycol, 1,2-propylene glycol, 1,4-butanediol, 1,3-butanediol, 1,5-pentanediol, 3-methyl-1,5-pentanediol, 1,6-hexanediol, 1,8-octanediol, 2,4-diethyl-1,5-pentanediol, 2-butyl-2-ethyl-1,3-propanediol, 2,2-diethyl-1,3-propanediol, dodecanediol, neopentyl glycol, trimethylol propane, pentaerythritol, dipentaerythritol and glycerin.

Examples of phenols include low molecular weight phenol compounds such as phenol, resorcinol, hydroquinone, pyrocatechol, bisphenol A, dihydroxydiphenylmethane (bisphenol F), bisphenol S, tetrabromobisphenol A, 1,3-bis (4-hydroxyphenyl) cyclohexane, 4,4'-dihydroxy-3,3'-dimethyldiphenylmethane, 4,4'-dihydroxybenzophenone, tris(4-hydroxyphenyl)methane, bis(4-hydroxyphenyl)ether, novolac phenol, novolac cresol, bis(3,5-dimethyl-4-hydroxyphenyl)sulfone, bis(4-hydroxyphenyl)sulfone and hydroxystyrene; and copolymers in which any of phenol novolac resins, cresol novolac resins, polyhydroxystyrene and hydroxystyrene is copolymerized with other copolymerizable vinyl monomer. Among them, the copolymer in which phenol novolac resin, cresol novolac resin, polyhydroxystyrene or hydroxystyrene copolymerized with other copolymerizable vinyl monomer is preferred. Examples of vinyl monomers copolymerizable with hydroxystyrene include (meth)acrylic esters such as methyl (meth)acrylate, ethyl (meth)acrylate, propyl (meth)acrylate, butyl (meth)acrylate, benzyl (meth)acrylate, and 2-ethylhexyl (meth)acrylate; unsaturated carboxylic acids and anhydrides thereof, such as (meth)acrylic acid, maleic acid, itaconic acid, maleic anhydride, and itaconic anhydride; monomers each having a hydroxyl group, such as 2-hydroxyethyl (meth)acrylate, 2-hydroxypropyl (meth)acrylate, and monoglycerol (meth)acrylate; monomers each having a nitrogen atom, such as (meth)acrylamide, (meth)acrylonitrile, diacetone (meth)acrylamide, and dimethylaminoethyl (meth)acrylate; monomers each having an epoxy group, such as allyl glycidyl ether and glycidyl (meth)acrylate; and styrene-based monomers such as styrene and a-methylstyrene. Herein, the term "(meth)acrylic acid" represents acrylic acid and methacrylic acid and the other derivatives also represent the compounds in the same manner.

Examples of the compound having a carboxyl group include polymers such as polyester resins, alkyd resins, and epoxy resins all of which have a carboxyl group; vinyl copolymers each having a carboxyl group; monocarboxylic acids such as formic acid, acetic acid, propionic acid, propiolic acid, butyric acid, isobutyric acid, hexanoic acid, heptanoic acid, octylic acid, nonanoic acid, isononanoic acid, decanoic acid, dodecanoic acid, stearic acid, benzoic acid, cinnamic acid, 2-naphthoic acid, nicotinic acid, isonicotinic acid, linseed oil fatty acid, tall oil fatty acid, soybean oil fatty acid, and dehydrated castor oil fatty acid; polyvalent carboxylic acids such as succinic acid, glutaric acid, adipic acid, azelaic acid, sebacic acid, dodecanedioic acid, compounds each having a decamethylene dicarboxyl group, phthalic acid, maleic acid, trimellitic acid, pyromellitic acid, tetrahydrophthalic acid, hexahydrophthalic acid, and methylhexahydrophthalic acid; hydroxycarboxylic acids such as lactic acid, citric acid, hydroxypivalic acid, 12-hydroxystearic acid, and malic acid; and α,β-unsaturated monomers each having a carboxyl group, such as acrylic acid, methacrylic acid, itaconic acid, mesaconic acid, maleic acid, and fumaric acid. Among these, vinyl copolymers each having a carboxyl group are preferred. Examples of the raw material in producing such a vinyl copolymer having a carboxyl group include (meth)acrylic esters such as methyl (meth)acrylate, ethyl (meth)acrylate, propyl (meth)acrylate, butyl (meth)acrylate, benzyl (meth)acrylate, and 2-ethylhexyl (meth)acrylate; unsaturated carboxylic acids and anhydrides thereof, such as (meth)acrylic acid, maleic acid, itaconic acid, maleic anhydride, and itaconic anhydride; monomers each having a hydroxyl group, such as 2-hydroxyethyl (meth)acrylate, 2-hydroxypropyl (meth)acrylate, and monoglycerol (meth)acrylate; monomers each having a nitrogen atom, such as (meth)acrylamide, (meth)acrylonitrile, diacetone (meth)acrylamide, and dimethylaminoethyl (meth)acrylate; monomers each having an epoxy group, such as allyl glycidyl ether and glycidyl (meth)acrylate; and styrene-based monomers such as styrene and α-methylstyrene. Polymers prepared by copolymerizing these monomers are preferably used as the compound having a carboxyl group.

The present invention will now be described in detail.
(1) Compound (I) and compound (IV)
   Compound (I) can be produced by, for example, allowing an alkenyl ether represented by general formula (III): 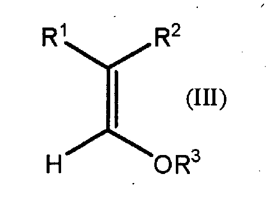 (wherein R¹, R², and R³ are the same as those defined above, respectively) to react with a hydrogen halide.
   The alkenyl ether represented by general formula (III) is commercially available or can be synthesized by a known process [for example, "Jikken Kagaku Kouza (Encyclopedia for Experimental Chemistry), Vol. 20, Yuuki Gousei (Organic synthesis) II alcohols and amines" edited by the Chemical Society of Japan, Fourth Edition, pp. 207-208, Maruzen Co., Ltd. (July 6, 1992)].
   Compound (IV) can be produced by, for example, allowing a dihydrofuran or dihydro-2H-pyran compound represented by general formula (VI): 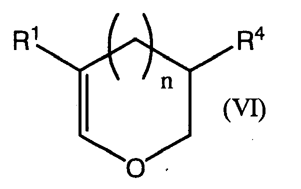 (wherein R¹, R⁴, and n are the same as those defined above, respectively) to react with a hydrogen halide.
   The dihydrofuran or dihydro-2H-pyran compound represented by general formula (VI) is commercially available or can be synthesized by a known process (for example, Journal of Organic Chemistry, 1998, Vol. 63, pp. 6007-6015 or Journal of Organic Chemistry, 1979, Vol. 44, pp. 364-368).
   As the hydrogen halide, a gaseous hydrogen halide, in particular, hydrogen chloride gas is preferably used. The amount of the hydrogen halide used is preferably 1 mole or more relative to 1 mole of the alkenyl ether represented by general formula (III) or the dihydrofuran or dihydro-2H-pyran compound represented by general formula (VI).
   The reaction temperature is preferably 0°C to 20°C.
   Compounds (I) and (IV) are useful as a protective agent for a hydroxyl group or a carboxyl group.
(2) Compound (II), compound (IIa), compound (V), and compound (Va)
   Compound (II) and compound (V) can be obtained by allowing a compound having a hydroxyl group to react with compound (I) or compound (IV), respectively. Compound (IIa) and compound (Va) can be obtained by allowing a compound having a carboxyl group to react with compound (I) or compound (IV), respectively.
   Compound (II) and compound (V) are derivatives obtained by substituting a hydroxyl group of the compound having the hydroxyl group with a group represented by general formula (II) or general formula (V), respectively. Compound (IIa) and compound (Va) are derivatives obtained by substituting a carboxyl group of the compound having the carboxyl group with a group represented by general formula (IIa) or general formula (Va), respectively. The ratio of the substitution of the hydroxyl group or the carboxyl group in compound (II), compound (IIa), compound (V), and compound (Va) is not particularly limited, but the ratio of the substitution is preferably 10 mole percent or more.
   The amount of compound (I) or compound (IV) used is not particularly limited. However, the amount used is preferably 1 to 10 moles, more preferably 1 to 5 moles, and still more preferably 2 to 4 moles relative to 1 mole of the hydroxyl group or the carboxyl group to be substituted (referred to as a group to be protected) in the compound having the hydroxyl group or the carboxyl group.
   The reaction temperature is preferably 0° C to 100° C, more preferably 0° C to 50° C, and still more preferably 0° C to 20° C.
   In the production process of the present invention, a base is preferably added. Examples of the base include, but are not particularly limited to, inorganic bases such as sodium hydroxide and potassium hydroxide; and organic bases such as ethylamine, diethylamine, and triethylamine. Among these, triethylamine is preferred. The amount of the base added is not particularly limited, but the amount is preferably 1 to 10 moles and more preferably 1 to 3 moles relative to 1 mole of compound (I) or compound (IV).
   In the production process of the present invention, an organic solvent may be used as needed. Examples of the organic solvent include hydrocarbon solvents such as hexane, toluene, and xylene; ether solvents such as dioxane and tetrahydrofuran; ketone solvents such as acetone, methyl ethyl ketone, and methyl isobutyl ketone; and aprotic polar solvents such as N,N-dimethylacetamide, N,N-dimethylformamide, and dimethyl sulfoxide. These solvents may be used alone or in combinations of two or more solvents.
   Since compound (II), compound (IIa), compound (V), and compound (Va) each have an acid-labile group represented by general formula (II), general formula (IIa), general formula (V), or general formula (Va), respectively, these compounds are useful as a component of a chemical amplification resist composition or the like. Among these, polyhydroxystyrene derivatives in which a hydroxyl group of a polyhydroxystyrene is substituted with a group represented by general formula (II) or general formula (V), phenol novolak resin derivatives in which a hydroxyl group of a phenol novolak resin is substituted with a group represented by general formula (II) or general formula (V), and the like are preferred. In the polyhydroxystyrene derivatives or the phenol novolak resin derivatives, all of the hydroxyl groups need not be substituted with the group represented by general formula (II) or general formula (V).
(3) Method for using protective agent for hydroxyl group or carboxyl group
   In the compound having a hydroxyl group or a carboxyl group, introduction of the group represented by general formula (II) or general formula (V), or general formula (IIa) or general formula (Va) and elimination of the group from the compound can be readily performed using compound (I) or compound (IV), respectively. Therefore, compound (I) or compound (IV) can be used as a protective agent for a hydroxyl group or a carboxyl group in organic syntheses.
   In the above, the group represented by general formula (II), general formula (IIa), general formula (V), or general formula (Va) can be converted into a hydroxyl group or a carboxyl group by heat treatment, an acid treatment, or the like.
   When the group represented by general formula (II), general formula (IIa), general formula (V), or general formula (Va) is converted into a hydroxyl group or a carboxyl group by heat treatment, the heat treatment is preferably performed at 160°C to 250°C.
   When the group represented by general formula (II), general formula (IIa), general formula (V), or general formula (Va) is converted into a hydroxyl group or a carboxyl group by an acid treatment, examples of the usable acid include sulfuric acid, hydrochloric acid, and p-toluenesulfonic acid. Among these, p-toluenesulfonic acid is preferred. The amount of the acid used is preferably 0.01 to 50 moles relative to 1 mole of the group represented by general formula (II), general formula (IIa), general formula (V), or general formula (Va). The temperature during the acid treatment is preferably 80°C to 160°C.
   Furthermore, a photoacid generator may be used instead of the acid, and thus the group represented by general formula (II), general formula (IIa), general formula (V), or general formula (Va) may be converted into a hydroxyl group or a carboxyl group by an acid generated upon irradiation of light.
   As described above, since introduction of the group represented by general formula (II), general formula (IIa), general formula (V), or general formula (Va) to the compound having a hydroxyl group of a carboxyl group and elimination of the group from the compound can be readily performed, compound (II), compound (IIa), compound (V), and compound (Va) are useful as a component of a chemical amplification resist composition or the like.
   When compound (II), compound (IIa), compound (V), and compound (Va) is used as a component of a chemical amplification resist composition or the like, the compound may be used in a form in which all the hydroxyl groups or the carboxyl groups in the compound having a hydroxyl group or a carboxyl group are converted into the groups represented by general formula (II) or general formula (V), or general formula (IIa) or general formula (Va), and a part of the converted groups may reconverted into hydroxyl groups or carboxyl groups.
(4) Chemical amplification resist composition
   The chemical amplification resist composition of the present invention contains a polyhydroxystyrene derivative (hereinafter may be referred to as "base polymer") in which a hydroxyl group of a polyhydroxystyrene is substituted with a group represented by general formula (II) or general formula (V), or a phenol novolak resin derivative (hereinafter may be referred to as "base polymer") in which a hydroxyl group of a phenol novolak resin is substituted with a group represented by general formula (II) or general formula (V) and a photoacid generator. The order of addition and the mixing process of the compound and the photoacid generator in the preparation of the composition are not particularly limited.

The weight-average molecular weight of the base polymer is preferably 1,000 to 100,000 and more preferably 1,000 to 50,000.

The chemical amplification resist composition of the present invention is preferably dissolved or dispersed in an organic solvent (for example, a hydrocarbon solvent such as hexane, toluene, or xylene; an ether solvent such as dioxane or tetrahydrofuran; a glycol ether solvent such as propyleneglycol monomethyl ether or propyleneglycol monomethyl ether acetate; or a ketone solvent such as acetone, methyl ethyl ketone, or methyl isobutyl ketone) and is then applied on a wafer by spin coating. In such a case, the organic solvent is preferably used in an amount of 0.5 to 100 parts by weight relative to 1 part by weight of the base polymer.

Subsequently, a heating step (prebaking step) is generally conducted in order to evaporate the organic solvent on the wafer. The heating temperature in the prebaking step is preferably 80°C to 130°C. The above base polymers are excellent in heat resistance, and thus are hardly decomposed at the heating temperature as described above. The wafer after evaporating the organic solvent is irradiated with light from an exposure apparatus. The light can be selected according to a desired exposure wavelength. Examples thereof include far-infrared rays; visible light; near-ultetraviolet rays such as g-line, h-line, and i-line; KrF excimer laser; ArF excimer laser; DUV; and EUV. An electron beam may also be radiated. An acid is produced at the irradiated area by decomposition of the photoacid generator, resulting in the reproduction of a hydroxyl group. The compound having the reproduced hydroxyl group is then removed with an alkaline solution during the development. Thus, a positive resist pattern is formed.

Examples of the photoacid generator which may be used include onium salt compounds, sulfone compounds, sulfonic acid ester compounds, diazosulfone compounds, disulfonylmethane compounds, sulfoneimide compounds, nitrobenzyl compounds, naphthoquinone diazide compound and the like.

Examples of the onium salt compound include iodonium salt, sulfonium salt, phosphonium salt, diazonium salt, ammonium salt, and pyridinium salt such as bis(4-tert-butylphenyl)iodonium trifluoromethanesulfonate, bis(4-tert-butylphenyl)iodonium nonafluorobutanesulfonate, bis(4-tertbutylphenyl)iodonium-2-trifluoromethyl benzenesulfonate, bis(4-tert-butylphenyl)iodonium-10-camphorsulfonate, bis(4-tert-butylphenyl)iodonium-p-toluenesulfonate, diphenyliodonium trifluoromethanesulfonate, diphenyliodonium nonafluorobutanesulfonate, diphenyliodonium-2-trifluoromethyl benzenesulfonate, diphenyliodonium-10-camphorsulfonate, diphenyliodonium-p-toluenesulfonate, triphenylsulfonium trifluoromethanesulfonate, triphenylsulfonium nonafluorobutanesulfonate, triphenylsulfonium-2-trifluoromethyl benzenesulfonate, triphenylsulfonium-10-camphorsulfonate, triphenylsulfonium-p-toluenesulfonate, 4-tert-butylphenyldiphenylsulfonium trifluoromethanesulfonate, 4-tert-butylphenyldiphenylsulfonium nonafluorobutanesulfonate, 4-tert-butylphenyldiphenylsulfonium-2-trifluoromethyl benzenesulfonate, 4-tert-butylphenyldiphenylsulfonium-10-camphorsulfonate, 4-tert-butylphenyldiphenylsulfonium-p-toluenesulfonate, 4-tert-butoxyphenyldiphenylsulfonium trifluoromethanesulfonate, 4-tert-butoxyphenyldiphenylsulfonium nonafluorobutanesulfonate, 4-tert-butoxyphenyldiphenylsulfonium-2-trifluoromethyl benzenesulfonate, 4-tert-butoxyphenyldiphenylsulfonium-10-camphorsulfonate, and 4-tert-butoxyphenyldiphenylsulfonium-p-toluenesulfonate.

Examples of the sulfone compound include β-ketosulfones, β-sulfonylsulfones, and α-diazo compounds thereof. Specific examples of the sulfone compound include phenacylphenylsulfone, mesitylphenacylsulfone, bis(phenylsulfonyl)methane and the like.

Examples of the sulfonic acid ester compound include alkylsulfonates, haloalkylsulfonates, arylsulfonates, iminosulfonates and the like. Specific examples thereof include benzoin tosylate, pyrogallol tristrifluorosulfonate, pyrogallol methanesulfonic acid triester, nitrobenzyl-9,10-diethoxyanthracene-2-sulfonate, α-methylolbenzoisocyanate, α-methylolbenzoin octanesulfonate, α-methylolbenzoin trifluoromethanesulfonate, and α-methylolbenzoin dodecylsulfonate.

Examples of the sulfoneimide compound include, N-(trifluoromethylsulfonyloxy)succinimide, N-(trifluoromethylsulfonyloxy)phthalimide, N-(trifluoromethylsulfonyloxy)diphenylmaleimide, N-(trifluoromethylsulfonyloxy)bicyclo[2.2:1]hept-5-ene-2,3-dicarboximide, N-(trifluoromethylsulfonyloxy)-7-oxabicyclo[2.2.1]hept-5-ene-2,3-dicarboximide, N-(trifluoromethylsulfonyloxy)bicyclo[2.2.1]heptane-5,6-dioxy-2,3-dicarboximide, N-(trifluoromethylsulfonyloxy)naphthylimide, N-(camphorsulfonyloxy)succinimide, N-(camphorsulfonyloxy)phthalimide, N-(camphorsulfonyloxy)diphenylmaleimide, N-(camphorsulfonyloxy)bicyclo[2:2.1]hept-5-ene-2,3-dicarboximide, N-(camphorsulfonyloxy)-7-oxabicyclo[2.2.1]hept-5-ene-2,3-dicarboximide, N-(camphorsulfonyloxy)bicyclo[2.2.1]heptane-5,6-dioxy-2,3-dicarboximide, N-(camphorsulfonyloxy)naphthylimide, N-(4-methylphenylsulfonyloxy)succinimide, N-(4-methylphenylsulfonyloxy)phthalimide, N-(4-methylphenylsulfonyloxy)diphenylmaleimide, N-(4-methylphenylsulfonyloxy)bicyclo[2.2.1]hept-5-ene-2,3-dicarboximide, N-(4-methylphenylsulfonyloxy)-7-oxabicyclo[2.2.1]hept-5-ene-2,3-dicarboximide, N-(4-methylphenylsulfonyloxy)bicyclo[2.2.1]heptane-5,6-dioxy-2,3-dicarboximide, N-(4-methylphenylsulfonyloxy)naphthylimide, N-(2-trifluoromethylphenylsulfonyloxy) succinimide, N-(2-trifluoromethylphenylsulfonyloxy)phthalimide, N-(2-trifluoromethylphenylsulfonyloxy)diphenylmaleimide, N-(2-trifluoromethylphenylsulfonyloxy)bicyclo[2.2.1]hept-5-ene-2,3-dicarboximide, N-(2-trifluoromethylphenylsulfonyloxy)-7-oxabicyclo[2.2.1]hept-5-ene-2,3-dicarboximide, N-(2-trifluoromethylphenylsulfonyloxy)bicyclo[2.2.1]heptane-5,6-dioxy-2,3-dicarboximide, N-(2-trifluoromethylphenylsulfonyloxy)naphthylimide, N-(4-fluorophenylsulfonyloxy)succinimide, N-(4-fluorophenylsulfonyloxy)phthalimide, N-(4-fluorophenylsulfonyloxy)diphenylmaleimide, N-(4-fluorophenylsulfonyloxy)bicyclo[2.2.1]hept-5-ene-2,3-dicarboximide, N-(4-fluorophenylsulfonyloxy)bicyclo[2.2.1]heptane-5,6-dioxy-2,3-dicarboximide, N-(4-fluorophenylsulfonyloxy)naphthylimide and the like.

The amount of the photoacid generator to be included is preferably 0.01 to 50 parts by weight, more preferably 0.1 to 30 parts by weight, and still more preferably 0.5 to 25 parts by weight per 100 parts by weight of the base polymer.

The chemical amplification resist composition of the present invention is excellent in stability during the prebaking step and storage stability in a long period of time, and has satisfactory lithography characteristics.

The present invention will now be described more specifically by examples, comparative examples, a reference example, and test examples.

### Best Mode for Carrying Out the Invention

The structural determination in the examples and the reference example and the calculation of the introduction ratio of a protective group in the examples and the comparative examples were performed by ¹H-NMR spectrum (400 MHz, equipment for the measurement: GSX-400 manufactured by JEOL, Ltd., solvent for the measurement: deuterated chloroform).

For the differential thermogravimetric analysis, a TG/DTA 6200 manufactured by Seiko Instruments Inc. was used, and the measurement was performed under a nitrogen atmosphere from 40°C to 400°C at a heating rate of 10°C/min.

The weight-average molecular weight was measured by gel permeation chromatography (GPC) under the following conditions.

### (Conditions for GPC analysis)

Equipment: HLC-8120GPC (manufactured by Tosoh Corporation)
Column: TSKgel SuperHM-M (manufactured by Tosoh Corporation) Mobile phase: tetrahydrofuran (flow rate: 0.5 mL/min)
Column oven: 40° C
Detector: RI [RI-8000 (manufactured by Tosoh Corporation)] (REFERENCE EXAMPLE 1) Synthesis of 1-chloro-1-methoxy-2-methylprdpane

1-Methoxy-2-methylpropene (8.61 g) was cooled to 5°C, and hydrogen chloride gas (3.65 g) was bubbled in the liquid over a period of one hour, thereby obtaining a clear and colorless liquid (12.2 g). The liquid was confirmed to be 1-chloro-1-methoxy-2-methylpropane by the ¹H-NMR spectrum.

### (EXAMPLE 1) Synthesis of 2-chloro-3-methyltetrahydrofuran

Under a nitrogen atmosphere, 38 mL of a hexane solution (1.6 mol/L) of n-butyllithium was added to a tetrahydrofuran (THF) solution (45 mL) of diisopropylamine (5.6 g) at -78°C, and the mixture was stirred for 20 minutes. Subsequently, a THF solution (50 mL) of γ-butyrolactone (5.2 g) was added dropwise to the mixture over a period of 30 minutes while the temperature was kept at -78°C, and the resulting mixture was further stirred for 20 minutes. Methyl iodide (17 g) was then added dropwise over a period of 15 minutes. After the dropwise addition, the reaction solution was stirred for 4 hours while the temperature was increased to -30°C. The internal temperature was returned to room temperature. Subsequently, the reaction solution was treated with a saturated aqueous solution (25 mL) of ammonium chloride and the resulting solution was extracted with ether (50 mL). The organic layer was washed with a saturated saline solution (20 mL) and was then dried with sodium sulfate. Subsequently, the organic layer was distilled under reduced pressure to prepare 3-methyltetrahydrofuran-2-one (4 g). Subsequently, 50 mL of a THF solution (1.0 mol/L) of diisobutylaluminum hydride was added to an ether solution (50 mL) of the 3-methyltetrahydrofuran-2-one (4 g) at -20°C, and the resulting mixture was stirred for 1.5 hours. The internal temperature was returned to room temperature and methanol (35 mL) was added to the mixture. A precipitated solid was separated by filtetration, and the filtetrate was concentrated to prepare 3-methyltetrahydrofuran-2-ol (4.6 g). To the resulting 3-methyltetrahydrofuran-2-ol (4.6 g), p-toluenesulfonic acid monohydrate (0.023 g) was added and the mixture was heated at 140°C to 150°C until a product was not generated by distillation. A 10% aqueous solution of sodium carbonate was added to the product removed by distillation and the residue, and the mixture was extracted with chloroform. The solvent was then distilled off, and a distillation under reduced pressure was performed to prepare 4-methyl-2,3-dihydrofuran (2.5 g). This product was cooled to 5°C, and hydrogen chloride gas (1.1 g) was bubbled in the liquid over a period of 20 minutes, thereby obtaining a clear and colorless liquid (3.5 g).

The liquid was confirmed to be 2-chloro-3-methyltetrahydrofuran (a mixture of two structural isomers) by the ¹H-NMR spectrum. ¹H-NMR δ 6.22 (1H, d, J = 3.9, one isomer), 5.97 (1H, s, one isomer), 4.28-4.10 (3H, m, 2H from both isomers, 1H from one isomer), 4.05-3.96 (1H, m, one isomer), 2.80-2.7,1 (1H, m, one isomer), 2.52-2.35 (2H, m, both isomers), 2.10-2.00 (1H, m, one isomer), 1.92-1.77 (1H, m, one isomer), 1.64-1.55 (1H, m, one isomer), 1.18 (3H, d, J = 6.5, one isomer), 1.06 (3H, d, J = 7.1, one isomer)

### (EXAMPLE 2) Synthesis of 2-chloro-3-methyltetrahydropyran

A clear and colorless liquid (4.0 g) was obtained by performing the same operations as those in Example 1 except that γ-butyrolactone (5.2 g) was changed to valerolactone (6.0 g). The liquid was confirmed to be 2-chloro-3-methyltetrahydropyran by the ¹H-NMR spectrum. ¹H-NMR δ 6.07 (1H, d, J = 2.9), 4.03-3.96 (1H, m), 3.82-3.75 (1H, m), 2.10-1.40 (5H, m), 0.94 (3H, d, J = 6.6)

### (EXAMPLE 3) Synthesis of 2-chloro-3,5-diethyltetrahydropyran

A mixture of 1-ethoxy-1-butene (127 g), 2-ethylacrolein (53 g), dihydroquinone (13 mg), and zinc chloride (850 mg) was stirred in an autoclave at 90°C for four hours. The mixture was washed with water and was then distilled under reduced pressure (100°C/3.3 kPa), thereby obtaining 2-ethoxy-3,5-diethyl-3,4-dihydro-2H-pyran (92 g). Subsequently, a 2% palladium-carbon catalyst (9 g) and ethanol (280 g) were added to the product, and the mixture was stirred for four hours in the autoclave at room temperature under a hydrogen pressure of 0.7 MPa. The catalyst was separated by filtetration, and the filtetrate was concentrated under reduced pressure to prepare 2-ethoxy-3,5-diethyltetrahydropyran (84 g). p-Toluenesulfonic acid monohydrate (0.4 g) was added to the resulting product and the mixture was heated at 140°C to 150°C until a product was not generated by distillation. A 10% aqueous solution of sodium carbonate was added to the product removed by distillation and the residue, and the mixture was extracted with chloroform. The solvent was then distilled off, and a distillation under reduced pressure (84°C/3.2 kPa) was performed to prepare 3,5-diethyl-3,4-dihydro-2H-pyran (45 g). This product was cooled to 5°C, and hydrogen chloride gas (11.7 g) was bubbled in the liquid over a period of one hour, thereby obtaining a clear and colorless liquid (56 g).

The liquid was confirmed to be 2-chloro-3,5-diethyltetrahydropyran by the ¹H-NMR spectrum. ¹H-NMR δ 6.13 (1H, d, J = 2.9), 3.77-3.70 (1H, m), 3.67-3.57 (1H, m), 1.98-1.13 (8H, m), 0.92 (6H, m)

### (EXAMPLE 4) Synthesis of phenol novolak resin derivative in which hydroxyl group is substituted with 1-methoxy-2-methylpropoxy group

A phenol novolak resin having a weight-average molecular weight of 10,000 (2.7 g) (manufactured by Showa Highpolymer Co., Ltd.) was dissolved in methyl ethyl ketone (17.5 mL). To the solution, 1-chloro-1-methoxy-2-methylpropane (11.7 g) was added and was completely dissolved by stirring. Subsequently, triethylamine (7.6 g) was added dropwise over a period of about 30 minutes under stirring. After the completion of dropwise addition, the resulting solution was stirred for about three hours as is. Subsequently, a 20-fold volume of pure water was added to the resulting solution, and the mixture was extracted with methyl isobutyl ketone. The solvent was distilled off, and the resulting product was then added dropwise to 1,000 mL of pure water to perform reprecipitation. Thus, a pale yellow solid (4.6 g) was obtained. The ¹H-NMR spectrum showed that 75 mole percent of the hydroxyl groups were substituted with 1-methoxy-2-methylpropoxy groups. The weight-average molecular weight of the target substance was 16,000.

### (COMPARATIVE EXAMPLE 1) Synthesis of phenol novolak resin derivative in which hydroxyl group is substituted with 1-methoxy-2-methylpropoxy group

A phenol novolak resin having a weight-average molecular weight of 10,000 (2.7 g) (manufactured-by Showa Highpolymer Co., Ltd.) was dissolved in methyl ethyl ketone (17.5 mL). 1-Methoxy-2-methylpropene (11.7 g) was added to the solution and was completely dissolved by stirring. Subsequently, p-toluenesulfonic acid (0.26 g) was added to the solution under stirring. After the addition, the resulting solution was stirred for about three hours as is. Subsequently, a 20-fold volume of pure water was added to the resulting solution, and the mixture was extracted with methyl isobutyl ketone. The solvent was distilled off, and the resulting product was then added dropwise to 1,000 mL of pure water to perform reprecipitation. Thus, a pale yellow solid (2.6 g) was obtained. The ¹H-NMR spectrum showed that the hydroxyl groups were not substituted with 1-methoxy-2-methylpropoxy group (the reaction was slow).

### (COMPARATIVE EXAMPLE 5) Synthesis of phenol novolak resin derivative in which hydroxyl group is substituted with 1-ethoxyethoxy group

A phenol novolak resin having a weight-average molecular weight of 10,000 (2.7 g) (manufactured by Showa Highpolymer Co., Ltd.) was dissolved in methyl ethyl ketone (17.5 mL). 1-Chloro-1-ethoxyethane (8.4 g) was added to the solution and was completely dissolved by stirring. Subsequently, triethylamine (7.6 g) was added dropwise over a period of about 30 minutes under stirring. After the completion of dropwise addition, the resulting solution was stirred for about three hours as is. The reaction solution was gradually colored and became black. Subsequently, a 20-fold volume of pure water was added to the resulting solution, and the mixture was extracted with methyl isobutyl ketone. The solvent was distilled off, and the resulting product was then added dropwise to 1,000 mL of pure water to perform reprecipitation. Thus, a brown solid (1.73 g) was obtained. The ¹H-NMR spectrum showed that 17 mole percent of the hydroxyl groups were substituted with 1-ethoxyethoxy groups and the product contained a polymer of ethyl vinyl ether.

### (EXAMPLE 5) Synthesis of polyhydroxystyrene derivative in which hydroxyl group is substituted with 1-methoxy-2-methylpropoxy group

A polyhydroxystyrene having a weight-average molecular weight of 20,000 (2.7 g) (manufactured by Aldrich Corporation) was dissolved in N,N-dimethylacetamide (17.5 mL). To the solution, 1-chloro-1-methoxy-2-methylpropane (11.7 g) was added and was completely dissolved by stirring. Subsequently, triethylamine (7.6 g) was added dropwise over a period of about 30 minutes under stirring. After the completion of dropwise addition, the resulting solution was stirred for about three hours as is. Subsequently, a 20-fold volume of pure water was added to the resulting solution, and the mixture was extracted with methyl isobutyl ketone. The solvent was distilled off, and the resulting product was then added dropwise to 1,000 mL of pure water to perform reprecipitation. Thus, a pale yellow solid (5.0 g) was obtained. The ¹H-NNR spectrum showed that 100 mole percent of the hydroxyl groups were substituted with 1-methoxy-2-methylpropoxy groups. The weight-average molecular weight of the target substance was 34,300.

### (COMPARATIVE EXAMPLE 3) Synthesis of polyhydroxystyrene derivative in which hydroxyl group is substituted with 1-ethoxyethoxy group

A polyhydroxystyrene having a weight-average molecular weight of 20,000 (2.7 g) (manufactured by Aldrich Corporation) was dissolved in N,N-dimethylacetamide (17.5 mL). 1-Chloro-1-ethoxyethane (8.4 g) was added to the solution and was completely dissolved by stirring. Subsequently, triethylamine (7.6 g) was added dropwise over a period of about 30 minutes under stirring. After the completion of dropwise addition, the resulting solution was stirred for about three hours as is. The reaction solution was gradually colored and became black. Subsequently, a 20-fold volume of pure water was added to the resulting solution, and the mixture was extracted with methyl isobutyl ketone. The solvent was distilled off, and the resulting product was then added dropwise to 1,000 mL of pure water to perform reprecipitation. Thus, a brown solid (1.3 g) was obtained. The ¹H-NMR spectrum showed that 10 mole percent of the hydroxyl groups were substituted with 1-ethoxyethoxy groups and the product contained a polymer of ethyl vinyl ether.

### (TEST EXAMPLE 1) Evaluation of thermal stability of protected polymer

A differential thermogravimetric analysis was performed using the phenol novolak resin derivatives produced in Example 4 and Comparative Example 2. Table 1 shows the measurement results of the temperature at which the weight loss is started.

**Table 1 Temperature at which weight loss is started**

| Example 4 | 202° C |
|---|---|
| Comparative Example 2 | 69°C |

The phenol novolak resin derivative produced in Example 4 was excellent in thermal stability as compared with the phenol novolak resin derivative produced in Comparative Example 2.

### (EXAMPLE 6) Preparation of chemical amplification resist composition

The solid (20 g) obtained in Example 5 was dissolved in propylene glycol monomethyl ether acetate (80 g). Diphenyliodonium trifluoromethanesulfonate (manufactured by Aldrich Corporation) (1 weight percent) was blended with the resulting solution to prepare a chemical amplification resist composition.

### (TEST EXAMPLE 2) Evaluation of chemical amplification resist composition

The sensitivity of the chemical amplification resist composition prepared in Example 6 was evaluated under the following conditions.

### (Conditions for exposure and development)

Prebake: 100°C × 5 min.
Exposure: Ultrahigh-pressure and low-pressure mercury lamps (254 nm) manufactured by Ushio Inc.
Exposure dose: 8 mJ/cm²
Post exposure bake: 120°C × 2 min.
Developer: 2.38 weight percent aqueous solution of tetramethylammonium hydroxide

When a resist pattern prepared under the above conditions was observed with a scanning electron microscope, resolution of a 2 µm line and space pattern was ascertained with the line width as specified.

### (EXAMPLE 7) Synthesis of polyhydroxystyrene derivative in which hydroxyl group is substituted with 3-methyl-2-tetrahydrofuranyloxy group

A polyhydroxystyrene having a weight-average molecular weight of 20,000 (2.7 g) (manufactured by Aldrich Corporation) was dissolved in N,N-dimethylacetamide (17.5 mL). To the solution, 2-chloro-3-methyltetrahydrofuran (11.5 g) was added and was completely dissolved by stirring. Subsequently, triethylamine (7.6 g) was added dropwise over a period of about 30 minutes under stirring. After the completion of dropwise addition, the resulting solution was stirred for about three hours as is. Subsequently, a 20-fold volume of pure water was added to the resulting solution, and the mixture was extracted with methyl isobutyl ketone. The solvent was distilled off, and the resulting product was then added dropwise to 1,000 mL of pure water to perform reprecipitation. Thus, a pale yellow solid (5.0 g) was obtained. The ¹H-NMR spectrum showed that 100 mole percent of the hydroxyl groups were substituted with 3-methyl-2-tetrahydrofuranyloxy groups. The weight-average molecular weight of the target substance was 34,000.

### (EXAMPLE 8) Synthesis of polyhydroxystyrene derivative in which hydroxyl group is substituted with 3-methyl-2-tetrahydropyranyloxy group

A polyhydroxystyrene having a weight-average molecular weight of 20,000 (2.7 g) (manufactured by Aldrich Corporation) was dissolved in N,N-dimethylacetamide (17.5 mL). To the solution, 2-chloro-3-methyltetrahydropyran (12.9 g) was added and was completely dissolved by stirring. Subsequently, triethylamine (7.6 g) was added dropwise over a period of about 30 minutes under stirring. After the completion of dropwise addition, the resulting solution was stirred for about three hours as is. Subsequently, a 20-fold volume of pure water was added to the resulting solution, and the mixture was extracted with methyl isobutyl ketone. The solvent was distilled off, and the resulting product was then added dropwise to 1,000 mL of pure water to perform reprecipitation. Thus, a pale yellow solid (5.1 g) was obtained. The ¹H-NMR spectrum showed that 100 mole percent of the hydroxyl groups were substituted with 3-methyl-2-tetrahydropyranyloxy groups. The weight-average molecular weight of the target substance was 36,300.

### (EXAMPLE 9) Synthesis of polyhydroxystyrene derivative in which hydroxyl group is substituted with 3,5-diethyl-2-tetrahydropyranyloxy group

A polyhydroxystyrene having a weight-average molecular weight of 20,000 (2.7 g) (manufactured by Aldrich Corporation) was dissolved in N,N-dimethylacetamide (17.5 mL). To the solution, 2-chloro-3,5-diethyltetrahydropyran (16.9 g) was added and was completely dissolved by stirring. Subsequently, triethylamine (7.6 g) was added dropwise over a period of about 30 minutes under stirring. After the completion of dropwise addition, the resulting solution was stirred for about three hours as is. Subsequently, a 20-fold volume of pure water was added to the resulting solution, and the mixture was extracted with methyl isobutyl ketone. The solvent was distilled off, and the resulting product was then added dropwise to 1,000 mL of pure water to perform reprecipitation. Thus, a pale yellow solid (5.7 g) was obtained. The ¹H-NMR spectrum showed that 100 mole percent of the hydroxyl groups were substituted with 3,5-diethyl-2-tetrahydropyranyloxy groups. The weight-average molecular weight of the target substance was 43,300.

### (EXAMPLE 10) Preparation of chemical amplification resist compositions

Each of the solids (20 g) obtained in Examples 7, 8, and 9 was dissolved in propylene glycol monomethyl ether acetate (80 g). Diphenyliodonium trifluoromethanesulfonate (manufactured by Aldrich Corporation) (1 weight percent) was blended with each solution to prepare a chemical amplification resist composition.

### (TEST EXAMPLE 3) Evaluation of chemical amplification resist compositions

The sensitivity of the three chemical amplification resist compositions prepared in Example 10 was evaluated under the following conditions.

### (Conditions for exposure and development)

Prebake: 100°C × 5 min.
Exposure: Ultrahigh-pressure and low-pressure mercury lamps (254 nm) manufactured by Ushio Inc.
Exposure dose: 8 mJ/cm²
Post exposure bake: 120°C× 2 min.
Developer: 2.38 weight percent aqueous solution of tetramethylammonium hydroxide

When three resist patterns prepared under the above conditions were observed with a scanning electron microscope, resolution of a 2 µm line and space pattern was ascertained in all the patterns with the line width as specified.

### Industrial Applicability

According to the present invention, a process for producing a compound having an acid-labile group with high reaction rate, less side reaction, or in a high yield; and the like can be provided.

## Claims

1. A process for producing a compound having a group represented by general formula (II): 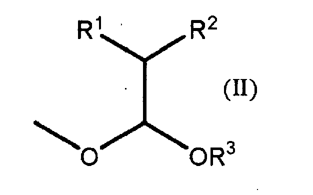 (wherein R¹, R², and R³ may be the same or different, and each represent a substituted or unsubstituted alkyl; a substituted or unsubstituted aryl, or a substituted or unsubstituted aralkyl, or R¹ and R² may bind to each other to form an alicyclic hydrocarbon ring together with the adjacent carbon atoms, or R² and R³ may bind to each other to form a heterocyclic ring together with the adjacent O-C-C that may have a substituent), which comprises allowing a compound having a hydroxyl group to react with a halogenated alkyl ether represented by general formula (I): 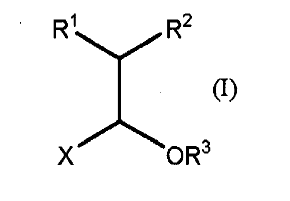 (wherein R¹, R², and R^{3'} are the same as those defined above, respectively and X represents a halogen atom).

2. A process for producing a compound having a group represented by general formula (IIa): 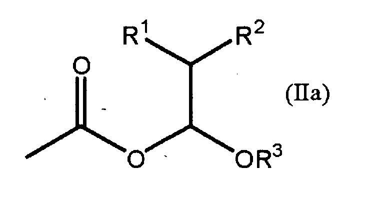 (wherein R¹, R², and R³ are the same as those defined above, respectively), which comprises allowing a compound having a carboxyl group to react with a halogenated alkyl ether' represented by general formula (I): 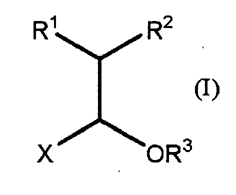 (wherein R¹, R², R³, and X are the same as those defined above, respectively).

3. A polyhydroxystyrene derivative wherein a hydroxyl group of the polyhydroxystyrene is substituted with a group represented by general formula (II): 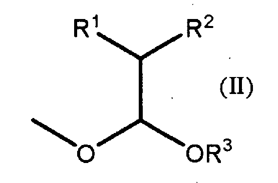 (wherein R¹, R², and R³ are the same as those defined above, respectively).

4. A phenol novolak resin derivative wherein a hydroxyl group of the phenol novolak resin is substituted with a group represented by general formula (II): 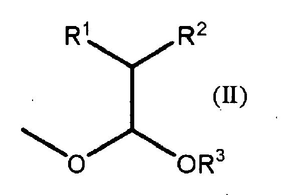 (wherein R¹, R², and R³ are the same as those defined above, respectively).

5. A protective agent for a hydroxyl group, which comprises a halogenated alkyl ether represented by general formula (I): 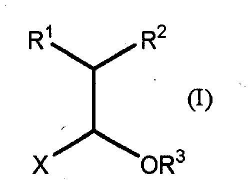 (wherein R¹, R², R³, and X are the same as those defined above, respectively).

6. A protective agent for a carboxyl group, which comprises a halogenated alkyl ether represented by general formula (I): 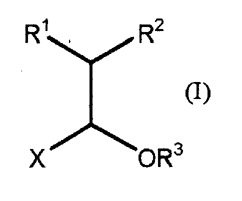 (wherein R¹, R², and R³ are the same as those defined above, respectively).

7. A chemical amplification resist composition which comprises a polyhydroxystyrene derivative in which a hydroxyl group of a polyhydroxystyrene is substituted with a group represented by general formula (II): 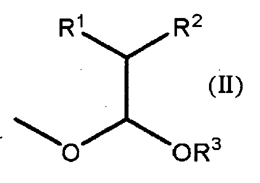 (wherein R¹, R², and R³ are the same as those defined above, respectively) and a photoacid generator.

8. A chemical amplification resist composition which comprises a phenol novolak resin derivative in which a hydroxyl group of a phenol novolak resin is substituted with a group represented by general formula (II): 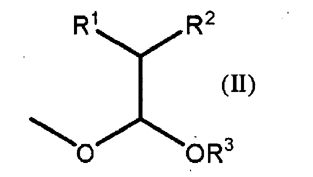 (wherein R¹, R², and R³ are the same as those defined above, respectively) and a photoacid generator.

9. A process for producing a compound having a group represented by general formula (V): 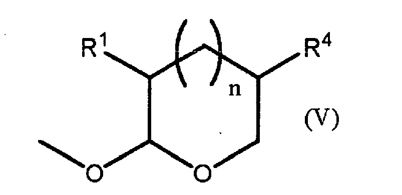 (wherein R¹ is the same as that defined above, R⁴ represents a hydrogen atom, a substituted or unsubstituted alkyl, a substituted or unsubstituted aryl, or a substituted or unsubstituted aralkyl, and n represents 0 or 1), which comprises allowing a compound having a hydroxyl group to react with a tetrahydrofuran or tetrahydropyran compound represented by general formula (IV): 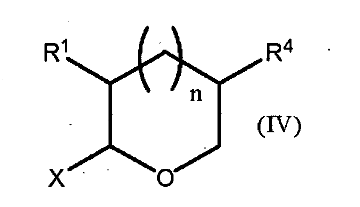 (wherein R¹, R⁴, n, and X are the same as those defined above, respectively).

10. A process for producing a compound having a group represented by general formula (Va): 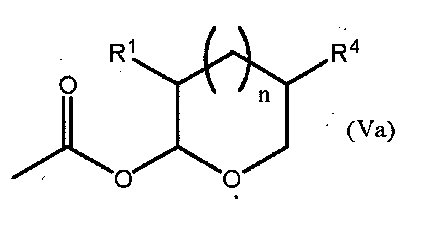 (wherein R¹, R⁴, and n are the same as those defined above, respectively), which comprises allowing a compound having a carboxyl group to react with a tetrahydrofuran or tetrahydropyran compound represented by general formula (IV): 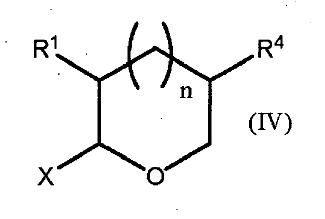 (wherein R¹, R⁴, and n are the same as those defined above, respectively).

11. A polyhydroxystyrene derivative wherein a hydroxyl group of the polyhydroxystyrene is substituted with a group represented by general formula (V): 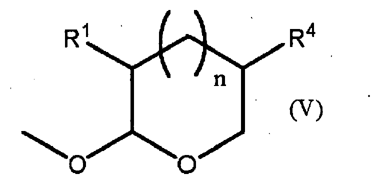 (wherein R¹, R⁴, and n are the same as those defined above, respectively).

12. A phenol novolak resin derivative wherein a hydroxyl group of the phenol novolak resin is substituted with a group represented by general formula (V): 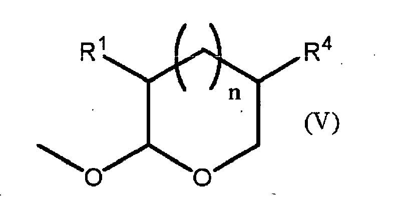 (wherein R¹, R⁴, and n are the same as those defined above, respectively).

13. A protective agent for a hydroxyl group, which comprises a tetrahydrofuran or tetrahydropyran compound represented by general formula (IV): 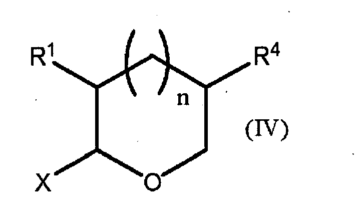 (wherein R¹, R⁴, and n are the same as those defined above, respectively).

14. A protective agent for a carboxyl group, which comprises a tetrahydrofuran or tetrahydropyran compound represented by general formula (IV): 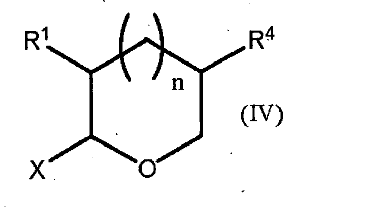 (wherein R¹, R⁴, and n are the same as those defined above, respectively).

15. A chemical amplification resist composition which comprises a polyhydroxystyrene derivative in which a hydroxyl group of a polyhydroxystyrene is substituted with a group represented by general formula (V): 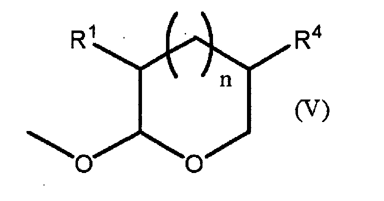 (wherein R¹, R⁴, and n are the same as those defined above, respectively) and a photoacid generator.

16. A chemical amplification resist composition which comprises a phenol novolak resin derivative in which a hydroxyl group of a phenol novolak resin is substituted with a group represented by general formula (V): 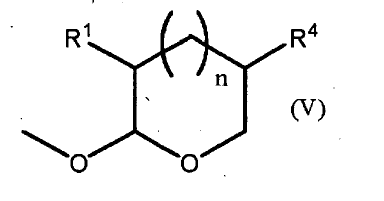 (wherein R¹, R⁴, and n are the same as those defined above, respectively) and a photoacid generator.

17. A tetrahydrofuran or tetrahydropyran compound represented by general formula (IV): 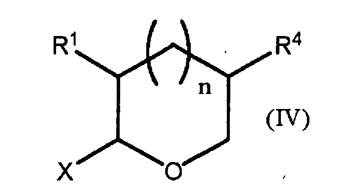 (wherein R¹, R⁴, and n are the same as those defined above, respectively).
